# EUROPEAN PATENT SPECIFICATION

(11) **EP 2 686 481 B1**
(45) Date of publication and mention of the grant of the patent: **12.07.2017**
(21) Application number: 12761451.9
(22) Date of filing: 15.03.2012
(51) Int. Cl.: D21G 3/00, B05C 11/04, B23D 35/00, B26B 9/00, B31F 1/14, B41F 9/10, B41N 10/00, C23C 14/48, D21H 23/34

(54) **BLADE SHAPED TOOL AND METHOD FOR ITS MANUFACTURING**
KLINGENFÖRMIGES WERKZEUG UND VERFAHREN ZU SEINER HERSTELLUNG
OUTIL EN FORME DE LAME ET SON PROCÉDÉ DE FABRICATION

(30) Priority: 18.03.2011 SE 1100200
(43) Date of publication of application: 22.01.2014
(73) Proprietor: PrimeBlade Sweden AB, 683 22 Sunne (SE)
(72) Inventor: LUNERFJORD, Alan, 686 91 Sunne (SE)
(74) Representative: Hynell, Magnus
(86) International application number: PCT/SE2012/050284
(87) International publication number: WO 2012/128700

(56) References cited:
- EP-A1- 0 908 309
- EP-A2- 0 327 530
- WO-A1-00/13860
- WO-A1-00/13860
- WO-A1-99/54520
- WO-A1-2005/059246
- US-A1- 2005 100 673
- US-A1- 2006 019 039
- US-A1- 2008 096 037

## Description

### TECHNICAL FIELD

The invention relates to a blade shaped tool belonging to that type of tools which comprises flat, strip shaped doctor and coater blades, which have a body of steel and at least one end portion, which is the working part of the tool during use thereof.

### BACKGROUND OF THE INVENTION

Blade shaped tools of many kinds are employed for various industrial applications. Doctor blades of the typed mentioned in the preamble are used in printing offices and printeries for scraping excess ink from an engraved printing or ductor roller. In the paper industry, coater blades, which also are made of strip steel of the above mentioned kind, are employed for scraping excess coating material from a running paper web in order to leave just an even layer of coating material of desired thickness on the web. Further, also crêping doctor blades for crêping a paper web should be mentioned in this connection. I all these cases, high quality requirements are raised on the employed blade shaped tools, which preferably are made of strip steel. They need to have, for their respective fields of use, an adequate thickness, width, length and shape, flexibility, hardness, and resistance to wear. Particularly, the combination of all these requirements have been difficult to satisfy.

The importance of wear resistance is elucidated in SE 519 466. This patent document suggests coating the doctor or coating blade electrolytically with a nickel layer containing wear resistant particles in order to improve the wear resistance of the blade. However, an electrolytically deposited nickel layer does not have a sufficient basic hardness in order to function satisfactorily for retaining the wear resistant particles. This is particularly true when the ink compositions which are used in those printing offices, or the coating compositions which are used in those paper industries, where the doctor blades or coater blades, respectively, are used, contain wearing particles of aluminium oxide, Al₂O₃ , which they normally do. The Al₂O₃-particles, which are extremely hard, namely "hollow out" the nickel layer and "carry away" the wear resistant particles from the nickel layer. This is at least a plausible reason why the working part of the tool blade is worn out too soon.

It has also been suggested, US 2005100673, to use a bar made of an austenitic stainless steel as a doctor element, which due to its shape has a sufficient stiffness and which is subjected to a gas plasma-assisted treatment, including implantation of foreign metal ions directly into the austenitic surface of the bar shaped doctor element. A product made of a bar of austenitic steel, however, regardless the surface hardness of the bar, has not a doctoring efficiency comparable with that of a doctor or coater blade, which, besides hardness and excellent wear resistance in the working part of the tool, has an adequate flexibility, which allows the tool to be pressed with a high pressure of contact, but at the same time resiliently, against a printing or ductor roller or against a running web. WO99/54520 discloses doctor or coater blades having PVD surface layers containing metal nitrides or carbides.

WO00/13860 discloses hardening of cutter knife edges by Plasma Ion Implantation of foreign ions comprising N, C, Mo, T or Ti.

Another prior art problem is the so called "burr", i.e. tiny projections which may develop on the trailing side of the tool, which may cause scratches or other damages on gravure printing cylinders and anilox rolls for flexographic printing, as well as defects on the web material, respectively, or other damages or defects of various kinds if the "burr" material would come loose.

### BRIEF DISCLOSURE OF THE INVENTION

It is the purpose of the present invention to address the above mentioned complex of problems in order to provide better blade shaped tools of the doctor and coater type of blade tools than according to prior art. Particularly, the invention aims at providing a thin, improved blade shaped tool which advantageously combines a high wear resistance with a high stiffness against bending, allowing the tool the be pressed with a high contact pressure against the object to be doctored or coated. This can be achieved therein that the invention is characterized in what is stated in the appending patent claims. Other characteristic features, objects and advantages of the invention will be apparent from the following detailed description and from the achieved results which also will be disclosed.

### BRIEF DESCRIPTION OF THE DRAWINGS

In the following description of the invention, reference will be made to the accompanying drawings, in which
Fig.1 shows a known profile of a doctor blade in the form of a blade shaped tool with rounded edges;
Fig. 1A shows in cross section an encircled and enlarged edge portion of the tool of Fig. 1 after wearing for a period of time;
Fig. 2 shows a known profile of a blade shaped tool with a beveled edge intended for doctor blades and coater blades;
Fig. 3 shows a known profile of a blade shaped tool with a lamella tip suitable for doctor blades;
Fig. 4 shows a known profile of a blade shaped tool with a cross ground edge for crêping doctor blades;
Fig. 5 shows schematically, enlarged and not according scale, the surface regions of the tool which have been encircled in Fig. 1, according to a first embodiment of the invention;
Fig. 6 shows, enlarged, in the same mode as in Fig. 5, the surfaced regions of the tool according to a second embodiment of the invention;
Fig. 7 shows a printing doctor blade, on which "burr" has been formed, i.e. tiny projections;
Fig. 8 schematically illustrates a simple device for testing the bending strength of a tool according to the invention;
Fig. 9 shows a picture made through scanning electron microscopy of a section of a doctor blade according to the invention, made of a low-alloy martensitic strip steel, showing in cross section a hard surface layer on a wide side of the blade and the carbide rich microstructure of the base material of the blade beneath the surface layer;
Fig. 10 illustrates the chemical characterization of the base material and of the surface layer of the sample shown in Fig. 9, employing energy-dispersive X-ray spectroscopy, EDS, image A showing the spectrum of the base material and image B showing the spectrum of the surface layer;
Fig. 11 shows the lighter layer which through scanning electron microscopy was observed on the surface of a tool made of a stainless, martensitic strip steel, which had been treated for 16 hours in a plasma-immersion-ion-implantation furnace, where the obtained surface layer appears to contain the same type of carbides as the steel base material beneath the surface but without the martensitic structure of the body,
Fig. 12 shows the same type of steel in the region of the surface and in the base material beneath the surface after 30 hours of treatment in the same furnace;
Fig. 13 is an atomic number contrast image which illustrates how the surface layer of a steel which has been bombarded by metal atoms with different atomic weight consists of two zones; an outer, darker zone which is enriched with lighter, implanted atoms, and an inner, lighter zone which is enriched with heavier atoms;
Fig. 14 shows the lighter, hard surface layer of a doctor blade made of a medium alloyed tool steel, and, beneath the surface layer, the carbide rich, martensitic microstructure of the tool steel;
Fig. 15 is an atomic number contrast image of the same tool steel as in Fig. 14, showing a light transition zone and light carbides which are rich in molybdenum and darker carbides which are rich in vanadium, which probably have been precipitated from existing retained austenite during the plasma-immersion-ion-implantation process, contributing to the hardness and bending resistance of the material; and
Fig. 16 shows a more conventional microstructure picture of a section of the same tool steel as in Fig. 14 and Fig. 15 with a typical martensitic structure, which contains dark needles of martensite, light lamella of cementite and tiny temper carbides, which have been precipitated from existing retained austenite during the plasma-immersion-ion-implantation process.

### DETAILED DESCRIPTION OF THE INVENTION

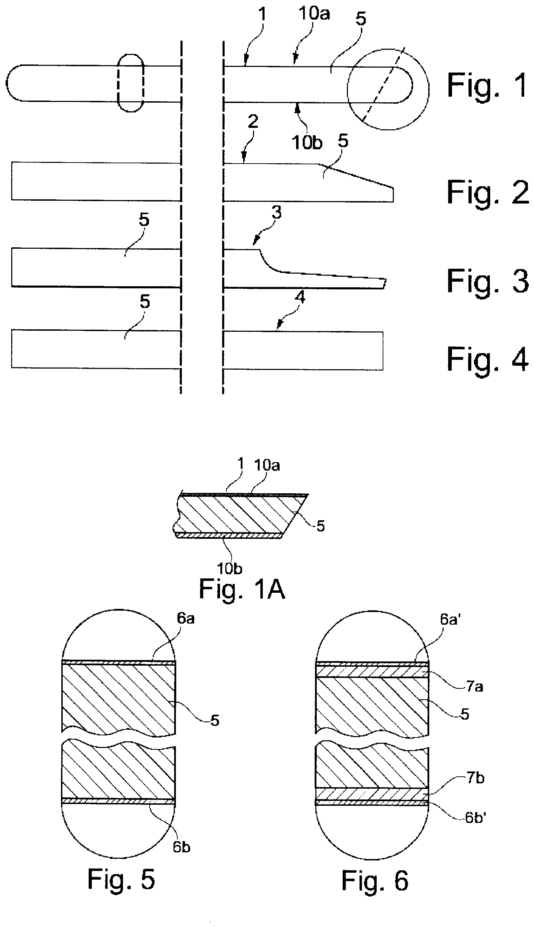

The four blade shaped tools according to the invention, whose profiles are shown in Fig. 1 - Fig. 4, are designated 1, 2, 3, and 4, respectively. As a base material in the tools, i.e. in the doctor blades, coater blades, or other tools according to the invention, there is used a hardenable, cold-rolled strip steel, for example any of those types of material which today conventionally are used for the manufacturing of doctor and/or coater blades, such as cold-rolled strips of carbon steel, low-alloyed hardenable steels or stainless, martensitic chromium steels. Among the large number of types of cold-rolled steel strips which presently are available on the market-place and which can be used as a base material for the blade shaped tools according to the invention, reference can be made to strip steels manufactured by BÖHLER-UDDEHOLM Precision Strip AB, Sweden, and SANDVIK AB, Sweden, which have nominal chemical compositions according to Table 1.

**Table**

| All percentages are weight-% | | | | | | | | | |
|---|---|---|---|---|---|---|---|---|---|
| Type | Trademark | C % | Si % | Mn % | Cr % | Mo % | V % | W % | Bal |
| Carbon steel | UHB 15LM | 0.75 | 0.2 | 0.73 | | | | | Fe |
| " | UHB 20C | 1.00 | 0.3 | 0.45 | | | | | " |
| " | UHB 20MC1 | 1.00 | 0.3 | 0.70 | 0.35 | | | | " |
| Alloyed but not stainless | Uddeholm MaxiBlade | 0.38 | 0.32 | 0.75 | 2.60 | 2.25 | 0.87 | | " |
| " | Uddeholm Hotvar | 0.55 | 1.0 | 0.75 | 2.6 | 2.25 | 0,85 | | " |
| " | Sanprint | 0.5 | 0.8 | 0.3 | 4 | 1.5 | | 2 | " |
| Low-alloyed | Sandvik 20C2 | 1.0 | 0.3 | 0.3 | 1.4 | | | | " |
| Stainless | Sandvik 13C26 | 0.65 | 0.4 | 0.65 | 13.0 | | | | " |
| " | Sandvik 7C27Mo2 | 0.38 | 0.4 | 0.6 | 13.5 | 1.0 | | | " |
| " | Sandvik C27 | 0.32 | 0.2 | 0.3 | 13.5 | | | | " |

The steels in the tools 1-4 have in their hardened and tempered condition a hardness between 500 and 700 HN (Hardness Vickers). The body 5 of the tools 1-4, i.e. their main portion, has a thickness which typically is between 0.07 and 0.4 mm as far as doctor blades is concerned, while coater blades typically has a thickness between 0.3 and 0.6 mm. The width is typically about 10 - 100 mm. The length can be up to about three to four meters, or more in exceptional cases, but may also, as far as doctor blades for printeries are concerned, be as short as 10 cm.

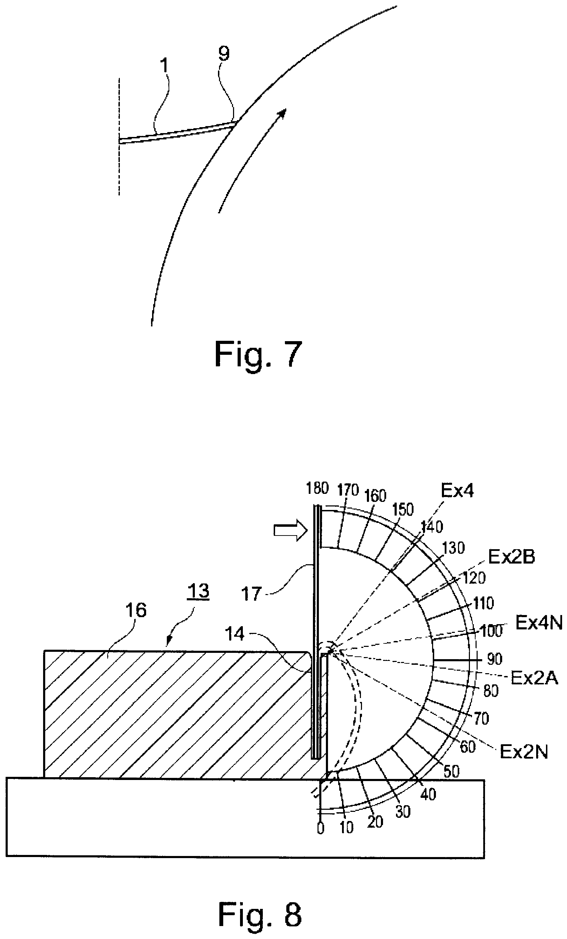

According to the invention, each of the two wide sides of the blade shaped tool has a surface layer 6a and 6b, respectively, Fig. 5, which contains hardness increasing products which are precipitated in the matrix of the steel near the surface although to a certain depth. All surfaces of the tool, according to the embodiment, are covered by such surface layers, which are very hard. It is particularly important that the two wide sides 10a and 10b are covered by the hard surface layers 6a, 6b, which are well integrated with the steel body 5, such that they will possess a high tensile strength. This, in combination with an adequate hardness of the body 5 of the tool, affords the tool a high bending strength according to elementary science of the strength of materials, which in turn makes it possible to press the tool with a high pressure of contact against e.g. a printing cylinder, without the tool being bent - deflected - to an unacceptable degree. It is also important that the hard surface layers 6a, 6b on the wide sides of the steel body extend as far as to the working edge of the tool in order to provide a good wear-resistance to the edge. On the other hand it is not important that the edge initially is covered by the same hard surface layer as the wide sides. Fig. 1A illustrates schematically the appearance of the working edge portion when its outermost tip - e.g. of the embodiment according to Fig. 1 - has been worn down. The material of the body 5 of the doctor blade 1 is exposed, but the very hard surface layers 6a and 6b, which contact the printing cylinder, reduce wear. The two hard surface layers also prevent that the edge portion of the doctor blade, which is pressed against the printing cylinder, is hollowed out or that material is pressed out by the cylinder to form burr 9, a phenomenon which is illustrated in Fig. 7. Burr impairs the quality of the printed product and is therefore a problem when doctor blades according to prior art are used, but also that problem can be eliminated through the present invention.

The surface layers 6a, 6b consist of components from the base material of the doctor blade, i.e. the strip steel which the doctor blade is made mainly of, and of hard products which have been incorporated in the surface layers. The hard products in said surface layers consist, according to the invention, mainly of hard products of reaction between, on one hand, one or more of the agents nitrogen, oxygen, and carbon and, on the other hand, one or more reactive metals that react with said agents, said agents and metals, respectively, having been implanted through plasma-immersion-ion- implantation. A Swedish terminology in the region ofplasma-immersion-ion-implantation has not yet been standardized. English is the common language, but even English has no terminology standards in this field. Presently, professionals use a number of definitions of different variants of the technique existing in this field, such as Plasma Immersion Ion Implantation (PIII), Plasma Immersion Ion Implantation and Deposition (PIIID), Metal Plasma Immersion Ion Implantation and Deposition (MePIII) and others, and also various combinations of said variants with their specific acronyms. Detailed information about this technique, which is employed according to the invention, can be found in "Handbook of Plasma Immersion and Deposition; Edited by André Anders". The base is the PIII-technique according to which the workpiece - as far as the invention is concerned, the workpiece is the steel strip/the tool that shall be treated - is placed directly in a plasma in a vacuum-chamber and is subjected to high, pulsating negative potential. When carrying out the method in practice, a large number of steel strips in the form of rolls are placed in the vacuum-chamber, each roll containing a steel strip having a length of one or two kilometers or more. The number of rolls may amount to the order often or twenty in each batch. A plasma sheath is formed around each of all these workpieces, even within the rolls between the winding layers, so that the pulsating potential causes ions to bombard all surfaces of every one of the strips. The process in other words is very rational. By employing one of the variants of this technique, on one hand nitrogen, oxygen and/or carbon atoms, and on the other hand metal atoms, preferably of one or more of the metals vanadium, molybdenum, tungsten, chromium, zirconium, titanium, and aluminum are caused to be ionized, i.e. to form ions, which bombard the workpieces/the steel strips, such that the reactive atoms are implanted in the steel, where they react with one another and possibly also with reactive agents which already exist in the steel material.

In the surface layers 6a, 6b of the tool 1, 2, 3, 4 according to the invention, Fig. 5, therefor hard products are formed, which may comprise or contain e.g. products generated through reactions between various implanted elements and/or between implanted elements and elements which exist originally in the steel, such as reaction products in the form of nitrides, carbides, and oxides, among them for example one or more of the following: titanium nitride (TiN) and aluminum nitride (A1N); vanadium carbide (VC), tungsten carbide (WC), molybdenum carbide (Mo2C/Mo6C) and chromium carbide (Cr3C2); and zirconium oxide (ZrO2), aluminum oxide (Al2O3) and chromium oxide (Cr2O3), respectively. Also mixed carbides as well as mixtures e.g. of type carbonitrides, such as titaniumcarbonitrides and others are conceivable and shall be included in the list of conceivable hardness promoting products, which may exist as discrete particles or as complex agglomerates of precipitated phases, such as new martensite and/or ferrite formed from retained austenite. It should be mentioned in this connection that the above list contains only a few selected but not restricting examples of hard reaction products which according to the invention may be implanted in the hard surface layers on the two wide sides of the tool.

According to a preferred embodiment of the invention, the surface layers 6a, 6b which contain said products which increase the hardness, are very thin. However, they have a thickness of at least 100 nm (nanometers), preferably at least 200 nm and as a maximum about 2 µm. Performed experiments have indicated that the thickness should be in the range 200 - 2000 nm. A preferred thickness may be at least 300 nm. The hardness of layers of material having so small thickness dimensions, however, can hardly be measured through conventional methods of hardness determination, such as for example Vickers. But it is possible to get an apprehension about the hardness through comparative studies of the bending strength of tools of the invention and of conventionally manufactured tools. Fig. 8 shows schematically a simple device 12 used for such studies, which give clear and distinct proofs of the improvements of bending strength which have been achieved according to the invention. These results also imply an important progress in the technique which is based on the use of doctor and/or coater blades. The improvement of the bending strength is particularly significant as it is combined with a pronounced improvement of the wear resistance of the tool which has been evidenced through field tests. When the bending strength of a strip shaped tool according to the invention shall be tested by means of the device 12, a first end portion 14 of a piece 13 of the strip is placed in a slot 15 in a holder 16, such that the opposite, second end portion 17 , which is longer than said first end portion 14, will stick up. The operator then folds down the second end to a stop, as is shown schematically in the drawing, suitably by hand power, as is indicated by an arrow, upon which he allows the test specimen to spring back. The degree of springback is a measure of the bending strength.

It is also possible to make an image of the structure and thickness of the surface layers by means of advanced microscopy, to some extent also their compositions, and in some cases their stratification in zones which have different contents of hard, implanted products.

Further, it has been possible to make a chemical characterization of the surface layers 6a, 6b and of the base material, i.e. of the body 5, by means of energy-dispersive X-ray spectroscopy, EDS.

According to a conceived development of the invention, there exist beneath the very hard but at the same time very thin surface layers 6a, 6b, Fig. 6, which contain said hardness increasing particles, underlying layers 7a, 7b which are harder than the base material of the body but not as hard as the surface layers but possibly thicker than them. More specifically, the underlying layers 7a, 7b may be characterized as nitrified layers, i.e. layers of steel containing a high content of nitrides, i.e. compounds of nitrogen and nitride forming metals which may exist in the steel alloy. These layers are established prior to the Plasma Immersion Ion Implantation of the or those metal/metals which in combination with carbon, nitrogen and/or oxygen form the hardness increasing products in the surface layers 6a, 6b. More specifically, the nitrified layers 7a, 7b according to this conceived development of the invention, are established through Plasma Immersion Ion Implantation of nitrogen in a surface layer of the tool at a temperature which may be lower than the tempering temperature of the steel, before the very thin surface layers 6a, 6b which contain said hardness increasing products are established in a subsequent process. However, nor may nitrogen penetrate into the steel to any great depth during a low temperature treatment of that kind, but through a thermal post-treatment at a higher temperature, but below the tempering temperature of the steel, the nitrogen may be caused to diffuse deeper into the steel. The diffusion may be enhanced by using a strip steel which has a high content of nitride forming metals, such as chromium, molybdenum, titanium, zirconium, vanadium and/or tungsten and others, wherein the nitrogen may diffuse to a depth of at least 0.1 µm, preferably to at least 0.5 µm (500 nm) already at a temperature of about 300 °C. Through selection of a steel having a tempering temperature which is not lower than about 500 °C, a thermal treatment at a temperature of up to 480 °C is conceivable in order to promote the diffusion of nitrogen to even greater depths and in any case to between at least 1 µm but not more than 10 µm, if so desired, but to maximum 30 % of the strip thickness, and thence also making the precipitation of metal nitrides in the steel more efficient. Therefore, in the nitride hardened layers 7a, 7b, the hardness may be increased with another 100-2500 HV-units (Hardness Vickers units) in excess of the base material's hardness of 500-700 HV. However, the nitrided layers exhibit a hardness gradient counted from the surface layers, because the amount of nitrogen which has diffused into the steel successively is lower and lower in the direction of diffusion. At least in a zone of the nitrided layers, however, the hardness should amount to between 1000 and 3000 HV. In this connection should be mentioned that thicker nitrided layers than 30 % of the thickness of the strip are not desired, because that would cause that the tool 1, 2, 3, 4, which typically is very thin, could be too brittle.

Now, hardness increasing products are established in an outermost surface layer of the nitreded and precipitation hardened layers 7a, 7b through Plasma Immersion Ion Implantation of one or more metals together with nitrogen, carbon and/or oxygen so that the hardness increasing products are formed as has been described in the foregoing. Fig. 6 schematically illustrates the result of the multi-step treatment. In thin surface layers 6a', 6b' which form part of the initially nitride hardened layers, now very hard particles in the form of Me-nitrides, oxides, and/or carbides are precipitated, where Me represents nitride, oxide, and/or carbide forming metals. Under the surface layers 6a', 6b', there are the said layers 7a and 7b, respectively, which have a thickness of at least 0.1 µm (100 nm), preferably at least 0.5 µm 500), and suitably at least 1 µm (1000 nm), however not more than 30 % of the thickness of the strip, and which contain precipitated nitrides of a type and in an amount which afford said layers an additional hardness, so that they at least in a zone of the layers attain a hardness of between 1000 and 3000 HV. The nitriding such as it has been described above has two desired effects. Firstly, it can contribute to the formation of a comparatively hard matrix for those particles which are implanted into the layers 6a', 6 b' in a subsequent step in order to increase the hardness still more, in which hard matrix the hard particles can be secured more safely. Secondly, the nitrided, comparatively thick layers 7a, 7b under the very thin surface layers 6a', 6b' form a support for the latter ones. Normally, very hard and very thin surface layers run a risk to be damaged if they rest like a thin skin directly on a much softer underlayer. According to the described embodiment of the invention, the very hard but thin surface layers 6a', 6b' are supported by the not that hard but thicker layer 7a and 7b, respectively, which reduces said risk. Then, under the nitrided layers 7a, 7b comes the steel material 5 which is hardened and tempered to a hardness of between 500 and 700 HV.

According to a conceivable development of the invention, no thermal treatment of the nitrided layers 7a, 7b is performed before the surface layers 6a', 6 b' are applied. Instead, the Plasma Immersion Ion Implantation of the surface layers 6a', 6b' is performed at an elevated temperature near although lower than the tempering temperature of the steel and for so long period of time that the desired diffusion of nitrogen into the steel will be achieved during this phase. According to a variant of this embodiment, a thermal treatment of the nitrided layers is performed before the surface layers 6a', 6 b' are applied, whereupon an additional thermal treatment takes place during a Plasma Immersion Ion Implantation of the surface layers 6a', 6b' at an elevated temperature.

Still another developed form of the invention is applicable in the cases when the strip steel is of a type which allows precipitation of secondary carbides, e.g. because the steel contains a substantial amount of retained austenite. Conventionally, cold rolled strip steels are hardened and tempered in a continuous process, which may cause some types of strip steel to contain a certain amount of retained austenite after hardening and tempering. The steel grades Sanprint (SANDVIK) and Uddeholm Hotvar, which are listed in Table 1, are examples of this type of steels which may make precipitation of secondary carbides during a second tempering operation possible, during which the retained austenite at least partly is transformed into secondary carbides and newly formed martenite. This is applied in said developed form of the invention according to any of the following procedures, namely
a) that the nitriding is performed through Plasma Nitriding at a temperature which provides a secondary tempering of the steel, including precipitation of secondary carbides and formation of newly formed fine martensite, at the same time as nitrogen diffuses into the steel strip and nitrides are precipitated, or
b) that the nitriding through Plasma Nitriding is not performed at a temperature which provides a secondary tempering according to a) but at a lower temperature, while instead the subsequent Plasma Immersion Ion Implantation of the surface layers 6a', 6b' is performed at a temperature which provides a secondary tempering of the steel, including precipitation of secondary carbides and formation of newly formed martensite, at the same time as nitrogen diffuses into the steel strip and nitrides are precipitated, or
c) that the nitriding is performed through Plasma Nitriding at a temperature which provides some but not a complete secondary tempering of the steel, including precipitation of secondary carbides and formation of newly formed fine martensite, at the same time as nitrogen diffuses into the steel strip and nitrides are precipitated as according to a), and that the subsequent Plasma Immersion Ion Implantation of the surface layers 6a', 6b' is performed at a temperature is performed at a temperature which provides a complementary and thence essentially complete secondary tempering of the steel, including precipitation of secondary carbides and formation of newly formed fine martensite, at the same time as the nitrogen also during this phase may diffuse into the steel strip and nitrides be precipitated.

Irrespective of which of the above procedures that is applied, there is obtained as a positive side effect that grain coarsening of the steel is counteracted.

According to still another conceivable development, the hard surface layers 6a, 6b, Fig. 5, or 6a', 6b', Fig. 6, may be coated with a sliding layer which reduces the friction against the roller, cylinder of corresponding which the doctor/coater blade is pressed against, e.g. a molybdenum layer which may be applied electrolytically according to any known technique, or a layer of Teflon (PTFE).

In the following, some examples of the invention shall be described and achieved results be reported from tests of some embodiments of doctor blades of the invention that have been performed.

### Example 1

In the example there was used as a doctor blade material a low-alloyed, cold-rolled, hardened and tempered strip steel of grade Sandvik 20C2 having the nominal chemical composition which is stated in Table 1. The strip thickness was 0.20 mm. The strip was ground to a profile according to Fig. 3. Ten rolls, each roll containing a strip with a length of 1 000 meters, were arranged to form a stack which was placed in a Vacuum Plasma Implantation and Condensation Furnace. In this furnace the work-piece, i.e. the strip material, was subjected to surface treatment through Ion Implantation and Condensation by Ion Discharging during a Plasmaphase. For the treatment in the furnace the following parameters applied:

| | |
|---|---|
| Pressure | 4 x 10⁻³ torr |
| Temperature | 160 °C |
| Duration of treatment | 16 hours |
| Frequence | 25 kHz |
| Doping metal | C, Mo, Ti, Cr, V |
| Gas | N₂ |

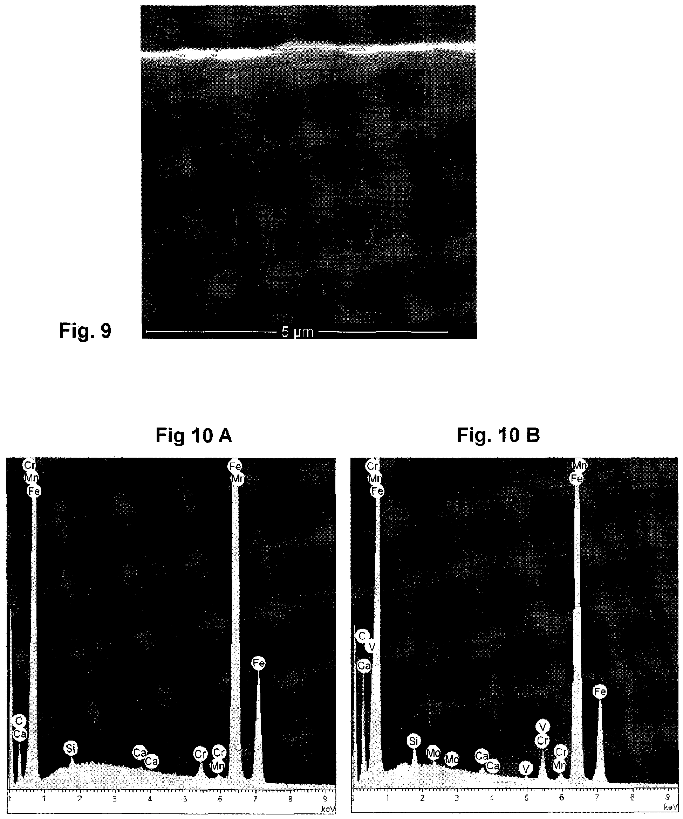

The microstructure of a sample of the surface treated strip material was studied by means of scanning electrone microscope. Fig. 9 shows the material greatly magnified. The lighter material a is the surface layer (designated 6a and 6b in the schematic drawing, Fig. 5), the thickness of which could be measured to about 350 nm. The microstructure of the base material b (designated 5 in Fig. 5), which contains major iron carbides minor carbides of not identified type, is visible under the surface layer. The hardness of the substrate, i.e. the base material or body 5, was measured to 595 HV.

The chemical characterization of the base material, Fig. 10A, and of a surface layer, Fig. 10B, was determined by energy-dispersive X-ray spectroscopy, EDS, which indicates the presence of different elements by measuring the energy of incoming X-ray quanta. The result of the investigation is visualized as a spectrum, in which the area of the peaks are related to the amount of the different elements. In both spectra, the elements iron, chromium, manganese and silicon of the base material dominate. The reason why the test indicates so high content of the base elements, however, may be that it cannot be avoided, due to the thinness of the surface layers, that also the underlying steel has influenced the test. In the surface layer, the right hand image B, however, there are, besides the elements which exist in the base material, also significant amounts of the implanted doping metals molybdenum and vanadium, and an increased amount of chromium which also was implanted. The test thus shows that the surface layer has been enriched on these elements, which in the surface layer have been combined with carbon and/or nitrogen to form very small carbides, nitrides and/or carbonitrides, the presence of which could not be detected by scanning electron microscopy. Indirectly, however, the presence of these hard products could be registered through the remarkable increase of the wear resistance of the tool that was achieved.

Thus, a doctor blade made in accordance with the invention of the surface treated strip material of the above mentioned steel grade, was subjected to a comparative field test in a flexo printing machine with a ceramic ink transferring plate cylinder, which was laser beam engraved to a line density of 220 lines/cm. When the plate cylinder had rotated a distance against the doctor blade according to the invention corresponding to about 2.2 x 10⁶ running meters (2 200 000 metres), the doctor blade was considered to be worn out according to the criteria which were valid in that printing office. Doctor blades of the same steel grade, manufactured according to conventional practice, i.e. in a mode including cold rolling, hardening and tempering, normally has a useful life corresponding to about 200 000 running meters in the same printing machine, using the same ink, in the same printing office. Thus, with the doctor blade of the invention, the working life span was extended eleven times, demonstrating a dramatic improvement of the tool's resistance to wear.

### Example 2

In this case there was used a stainless, martensitic chromium steel of grade Sandvik 13C26 but containing a somewhat lower content of silicon and manganese than according to Table 1; 0.3 Si and 0.55 Mn. The pretreatment was the same as in the foregoing example; cold-rolling to 0.2 mm thickness, hardening, tempering, and grinding to the profile shown in Fig. 3. Also this material was then subjected to Plasma Immersion Ion Implantation in the same furnace as in the foregoing example with the following parameters being applied for the treatment in the furnace.

| | |
|---|---|
| Pressure | 4 x 10⁻³ torr |
| Temperature | 300 °C |
| Duration of treatment | A first batch A treated in the furnace for 16 hours and second batch B for 30 hours |
| Frequence | 25 kHz |
| Doping metal | C, Mo, Ti, Cr, V |
| Gas | N₂ |

The purpose was to evaluate the importance of the duration of the treatment of the strip material in the furnace with reference to achieved springiness in terms of springback capacity of the doctor blade. It was judged that the bending test which has been described above with reference to Fig. 8 should be an adequate, comparative method for obtaining a comprehension about the importance of the duration of treatment. A first batch of strips, test A, was therefor treated for 16 hours, while a second batch of strips of the same kind, test B, was treated for 30 hours but in other respects in the same mode as the first batch. When subjected to the bending test, a test specimen from the first batch A sprung back to a permanent angle of about 85° , Ex 2A, while a specimen from the second batch B sprung back to a permanent angle of about 120°, Ex 2B. The starting material, i.e. the same type of normal doctor blade material, which had not been subjected to any Plasma Immersion Ion Implantation sprung back to only about 63°, Fig. 8, Ex 2N. This simple test thus indicates that the tool material according to the invention not only has an improved bending strength but also a good ductility, when compared with the conventional material, if ductility is defined as ability to retain strength and freedom from cracks when shape is altered.

The test also demonstrates that a prolonged duration of treatment, and thence probably an increased amount of hard products in the doctor blade material, gives a significant improvement of a feature which is very important for a strip shaped doctor blade, namely a high bending strength in combination with an adequate flexibility and ductility.

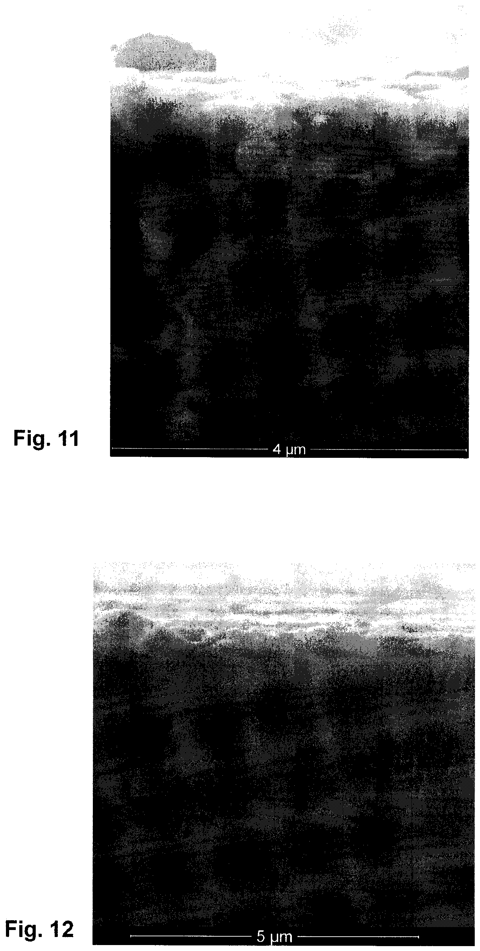

The two materials were also studied in a scanning electron microscope and by means of energy-dispersive X-ray spectroscopy, EDS. The microscope-photographic images, Fig. 11 and Fig. 12 (after 16 and 30 hours treatment, respectively, in the Plasma Immersion Ion Implantation furnace), are made so that also the surface of the specimens on top of the surface layers are viewed. The darker parts represent the base material, i.e. the steel in the tool body under the surface layers. There, one can, especially in Fig. 11, see clearly the typical microstructure of the stainless steel in the base material/the body with larger chromium carbides and smaller, unidentified carbides. The thicknesses after 16 and 30 hours treatment were measured to about 320 nm and 590 nm, respectively. The EDS-analysis gave the same information as in Example 1, namely that Mo, V, and Cr were enriched in the surface layers. The hardness of the base material (body 5) was measured to 580 HV after 16 hours as well as after 30 hours of treatment.

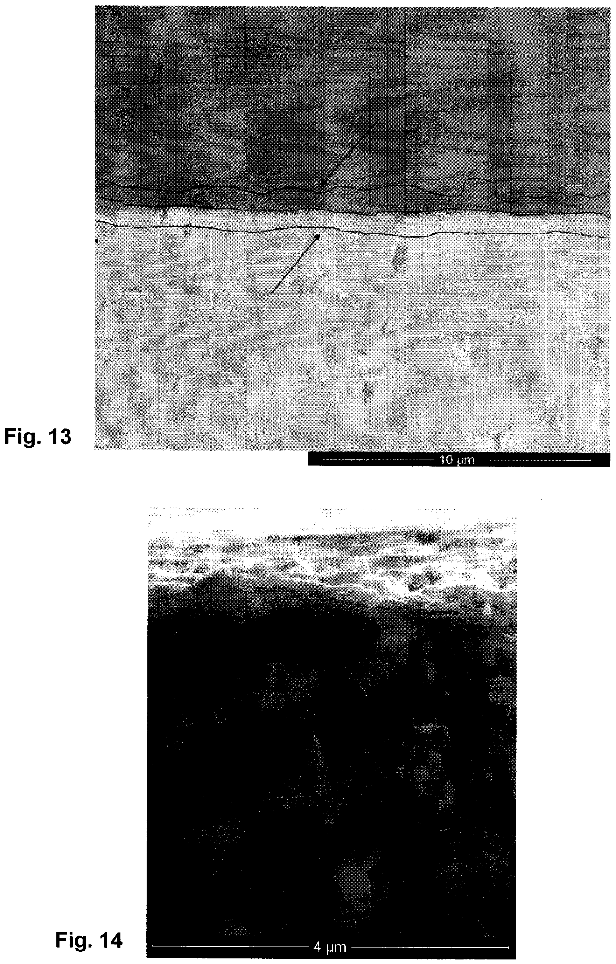

The microstructure of the surface layers could not be determined by means of scanning electron microscopy, but when applying atomic number contrast imaging, the layer which is farthest out, which is indicated by the upper arrow on the image, Fig. 13, became dark. This means that that outer layer has a lower mean atomic weight, which in turn means that the lighter atoms Ti and V, i.e. the doping metals titanium and vanadium have been enriched in that layer in the form of carbides. Under the darker layer one can discern a transition zone which is lighter and indicated by the lower arrow. It is assumed that this may indicate that this zone is enriched on the heavier doping metal molybdenum, and in that case probably in the form of M6C-carbides and/or -nitrides. Provided these assumptions are true, the stratification implies that there has been established an outer, very hard zone, which contains i.a. extremely hard titanium-carbides and/ or -nitrides, and an inner, not as hard zone, which contains not so extremely hard molybdenum-carbides and/or -nitrides. In other words, already in the surface layer there may be what can be termed de-escalation or phasing out of hardness, which is a desirable effect.

### Example 3

As a doctor blade material in this case there was used an alloyed but not stainless, cold-rolled, hardened, and tempered, strip-shaped tool-steel with the following nominal composition in weight-%: 0.55 C, 1.0 Si, 0.75 Mn, 2.6 Cr, 2.25 Mo, 0.85 V, balance iron and normally existing impurities. Also this strip material had a thickness of 0.2 mm and was ground to the same profile, Fig. 3, as in Examples 1 and 2. The material was treated in the same furnace and at the same pressure, period of time, frequency, and with the same gas, N₂, as in Example 1, but the temperature was higher, 400 °C, and as doping metals there were in this case used Mo, Ti, Cr, V, and B. Through the treatment there was established an about 380 nm thick surface layer; the light layer in Fig. 14. Also the surface of the layer is visible on the image. Under the layer, the carbide-rich microstructure of the tool-steel is visible.

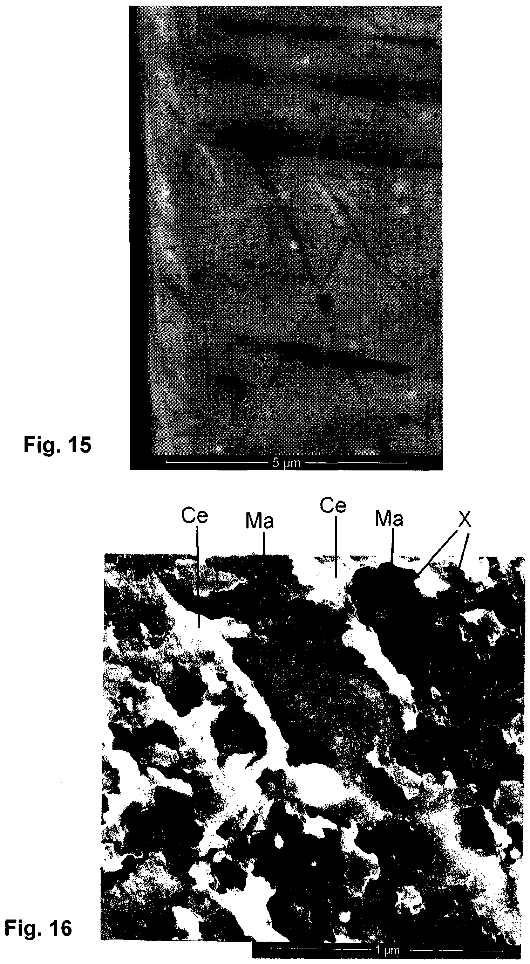

Fig. 15 shows an atomic number contrast image of the same treated steel. One can see on the image a light transition zone and molybdenum-rich carbides of M6C-type in the form of round particles, which are white on the image, and vanadium-rich carbides of MC-type which are dark, because their mean atomic number is low.

Fig. 16 shows a section of the base material, which has a typically martensitic structure. Adjacent to needles of martensite Ma there are lamella of cementite Ce, which have been precipitated during the tempering of the steel, and also small tempering carbides X, which have been precipitated from existing retained austenite during the Plasma Immersion Ion Implantation treatment.

### Example 4

The purpose of this example what is to illustrate the importance of performing the Plasma Immersion Ion Implantation in accordance with a preferred embodiment of the invention, in which the treatment in a first step is performed with nitrogen in the furnace but without any doping metals, while in a second step also doping metals are employed.

The same type of alloyed but not stainless tool steel was used as in Example 3. Also the pre-treatment of the strip material was the same; cold-rolling, hardening, and tempering. The strip which had been pre-treated in that way, was subjected, in two steps, to Plasma Immersion Ion Implantation treatment in the same furnace as according to the foregoing examples. In the two steps, the following parameters for the treatment in the furnace were applied:

| | Step 1 | Step 2 |
|---|---|---|
| Pressure | 4 x 10⁻³ torr | 4 x 10⁻³ torr |
| Temperature | 400 °C | 140 °C |
| Duration of treatment | 10 hours | 16 hours |
| Frequence | 25 kHz | 25 kHz |
| Doping metal | None | Mo, Ti, Cr, B, B |
| Gas | N₂ | N₂ |

In bending tests, which were performed in the same way as in tests which have been described in the foregoing, the test sample sprung back 140°, Ex 4, Fig.8. A test sample of the same steel grade in a cold-rolled, hardened and tempered condition but not subjected to Plasma-Immersion-Ion-Implantation sprung back 100°, Ex 4N, Fig.8. The two-step treatment thus provided a material, which had the best springiness of the tested materials, but a sample of this tool steel on the other hand had a comparatively good bending strength already in its normal condition, Ex 4N. The greatest improvement of the bending resistance was gained through the long term treatment of the stainless steel, Ex 2B, Fig. 8.

It should in this connection also be noted that in no case any crevices were observed in the hard surfaces layers of the tools according to the invention, neither in connection with the field tests nor in connection with the bending tests. In the surface layers, thus a high hardness is combined with a good toughness. While the hard hardness may be attributed to the hard products which have been provided in the surface layers, the good toughness of the surface layers - without binding the invention to any specific theory - depend on the fact that the surface layers are integrated with the original base material, an opinion which is supported by the results from energy dispersive X-ray spectroscopy (EDS) studies, but also because the hardness decreases successively from the very outside of the wide side surfaces of the tools towards the centre, without abrupt steps where high tensions could be concentrated.

In summary, a number of conclusions can be drawn from the experiences from the manufacturing of a number of different tool materials according to the invention, employing strip steels of different chemical compositions, and from a number of tests of the manufactured materials, namely:
that the hard surface layers contain - for the achievement of desired hardness, wear resistance, bending strength and ductility - hard products of reaction between on one hand one or more of the elements nitrogen, oxygen, and carbon, and on the other hand one or more metals which may react with said elements, which elements and metals, have been implanted into the surface layers of the steel strip through Plasma Immersion Ion Implantation, and
that the hard surface layers have a thickness of at least 100 nm (nanometer), preferably at least 200 nm and suitably at least 300 nm. The experiences also show that the thickness may as great as 2 000 nm.

## Claims

1. A strip shaped tool (1, 2, 3, 4) belonging to that type of tools which comprises flat, strip shaped doctor blades and coater blades, which have a body (5) of steel of a type belonging to the group of hardenable steels, which consists of carbon steels, low-alloyed steels, alloyed but not stainless steels, and stainless, martensitic chromium steels, said body having
two flats sides, which are covered by a hard surface layer (6a, 6b) which contains on one hand one or more of the elements nitrogen, oxygen, and carbon, and on the other hand one or more of with said elements reactive metals, and at least one end portion, which is the working part of the tool during use thereof, said one or more of the elements nitrogen, oxygen and carbon, and said one or more reactive metals, which comprise one or more of the metals titanium, tungsten, chromium, molybdenum, boron, vanadium, aluminum, and zirconium, exist in said surface layers as hardness promoting reaction products comprising particles of at least any of those compounds which belong to that group of compounds which consist of titanium nitride (TiN) and aluminum nitride (AlN), vanadium carbide (VC), tungsten carbide (WC) and chromium carbide (Cr₃C₂), and zirconium oxide (ZrO₂), aluminum oxide (Al₂O₃) and chromium oxide (Cr₂O₃), **characterized in that** that said hard surface layers (6a', 6b') cover a second, underlying layer (7a,7b) which contains nitrides precipitated in said second layer, increasing the hardness in at least a zone of said second layer to a hardness which is 100 - 1500 HV (Hardness Vickers) larger than the hardness in the steel material (5) under said second layer, and
that said second, underlying layer (7a, 7b) has a thickness of at least 0,1 µm (100 nm) but not more than 10 µm (0,010 mm),
that said second, underlying layers and said hard surface layers have microstructures of the types which are obtainable through Plasma Immersion Ion Implantation of nitrogen into the steel material, and subsequently, Plasma Immersion Ion Implantation of one or more of the reactive metals titanium, tungsten, chromium, molybdenum, boron, vanadium, aluminum, and zirconium together with one or more of the elements nitrogen, oxygen and carbon, into the steel,
that the body consists of a cold-rolled strip of steel which is hardened and tempered to a hardness between 500 and 700 HV (Hardness Vickers) in its hardened and tempered condition, and
that the body has a thickness between 0.07 and 0.6 mm.

2. A tool according to claim 1, **characterized in that** at least one zone of said second layer has a hardness between 1000 and 3000 HV.

3. A tool according to claim 1, **characterized in that** said second, underlying layer has a thickness of at least 0,5 µm (500 nm) but not more than 10 µm (0,010 mm).

4. A tool according to claim 1, **characterized in that** said second, underlying layer (7a,7b) has a thickness of at least 1 µm (1000 nm) but more than 10 µm (0,010 mm).

5. A tool according to claim 1, **characterized in that** the hard surface layers (6a, 6b) which contain hard products of reaction between on one hand one or more of the elements nitrogen, oxygen, and carbon, and on the other hand one or more metals, have a thickness of at least 100 nm (nanometers), preferably at least 200 nm.

6. A tool according to claim 5, **characterized in that** the hard surface layers have a thickness of at least 300 nm.

7. A tool according to any of claims 5 and 6, **characterized in that** at least two metals are implanted in the hard surface layers, including at least one first metal which is considerably lighter than a different, second metal, i.e. has a considerably lower atomic number than the second metal, said first, lighter metal being enriched in an outer zone of the hard surface layers, while said second, heavier metal is enriched in an inner zone of the hard surface layers.

8. A tool according to claim 7, **characterized in that** the first, lighter metal is any of the elements boron, aluminum, titanium, vanadium, and chromium, while the second, heavier metal is any of the elements zirconium, niobium, molybdenum, tantalum, and tungsten.

9. A tool according to any of claims 7 and 8, **characterized in that** each zone has a thickness of 0,1 - 1 µm.

10. A tool according to any of the preceding claims, **characterized in that** the tool's steel body between the hard surface layers has a predominantly martensitic structure, which contains major aggregations of precipitated cementite and very small carbide particles.

11. A tool according to claim 10, **characterized in that** the cementite is a primary phase which has been precipitated prior to the Plasma Immersion Ion Implantation, while at least a substantial portion of the very small carbide particles have been precipitated through transformation of retained austenite during said Plasma Immersion Ion Implantation.

12. Method of manufacturing a blade shaped tool (1, 2, 3, 4) according to claim 1, **characterized by** providing a strip-shaped body (5) of a hardenable steel which is cold-rolled, hardened, and tempered to a hardness between 500 and 700 HV (Hardness Vickers), and further **characterized in that** the tool is nitrided through Plasma Immersion Ion Implantation of nitrogen without any accompanying doping material at a temperature below the tempering temperature of the steel, causing precipitation of nitrides in a layer (7a,7b) under the surfaces of the flat sides of the tool on the , and
that a surface layer (6a', 6b') subsequently is caused to cover the nitrided layer (7a,7b) on the flat sides of the tool through Plasma Immersion Ion Implantation of on one hand one or more of said elements nitrogen, oxygen and carbon, and on the other hand one or more of the metals vanadium, molybdenum, tungsten, chromium, zirconium, titanium, boron, and aluminum, which elements and metals, respectively, form reaction products in the steel.

13. Method according to claim 12 **characterized in that** said subsequent Plasma Immersion Ion Implantation is performed at a temperature of 130 °C or higher, however below the tempering temperature of the steel, causing a thermal treatment of the nitrided layer for so long period of time that nitrogen is caused to diffuse further into the steel and nitrides be precipitated at a deeper level, so that said underlying layers (7a,7b) thence obtain a thickness of at least 0,1 µm (100 nm), preferably at least 0,5 µm (500 nm), and suitably at least 1 µm (1000 nm), however not more that 30 % of the thickness of the tool.

14. Method according to claim 12, **characterized in that** the introduction of said hard products of reaction between on one hand one or more of the elements nitrogen, oxygen and carbon, and on the other hand one or more of said metals which have a capacity to react with one or more of said elements, is performed through Plasma Immersion Ion Implantation and is caused to proceed for so long period of time that the reaction products, whose penetration depth is related to the period of time during which the Plasma Immersion Ion Implantation process is taking place, will penetrate into the steel to a depth corresponding to the thickness of the surface layer of at least 100 nm (nanometer), preferably at least 200 nm.

15. Method according to claim 12, **characterized in that**, when the starting material for the Plasma Immersion Ion Implantation is a refined, i.e. low-alloyed carbon steel in the form of a strip which has been cold-rolled, hardened and tempered to a hardness between 500 and 700 HBV, the steel strip is subjected to Plasma Immersion Ion Implantation for at least ten hours in a furnace atmosphere having a temperature of 130 - 170 °C.

16. Method according to claim 12, **characterized in that**, when the starting material for the Plasma Immersion Ion Implantation is an alloyed but not stainless hardenable steel in the form of a strip which has been cold-rolled, hardened and tempered to a hardness between 500 and 700 HV, the steel strip is subjected to Plasma Immersion Ion Implantation for at least ten hours in a furnace atmosphere having a temperature of 200 - 400 °C.

17. Method according to claim 12, **characterized in that**, when the starting material for the Plasma Immersion Ion Implantation is a stainless hardenable chromium steel in the form of a strip which has been cold-rolled, hardened and tempered to a hardness between 500 and 700 HV, the steel strip is subjected to Plasma Immersion Ion Implantation for at least ten hours in a furnace atmosphere have a temperature of 150 - 300 °C.

## Patentansprüche

1. Streifenförmiges Werkzeug (1, 2, 3, 4), das zu der Art von Werkzeugen gehört, die flache, streifenförmige Abstreifmesser und Streichmesser umfasst, die aufweisen einen Körper (5) aus Stahl, der zu einer Art gehört, der zu der Gruppe der härtbaren Stähle gehört, die besteht aus Kohlenstoffstählen, niedriglegierten Stählen, legierten aber nicht Edelstählen, edlen, martensitischen Chromstählen, wobei der Körper aufweist
zwei flache Seiten, die durch eine harte Oberflächenschicht (6a, 6b) bedeckt sind, die einerseits eines oder mehrere der Elemente Stickstoff, Sauerstoff und Kohlenstoff und andererseits eines oder mehrere von mit diesen Elementen reaktiven Metallen enthält, und wenigstens einen Endabschnitt, der den funktionalen Teil des Werkzeugs während dessen Gebrauch darstellt, wobei das eine oder die mehreren der Elemente Stickstoff, Sauerstoff und Kohlenstoff und das eine oder die mehreren reaktiven Metalle, die eines oder mehrere der Metalle Titan, Wolfram, Chrom, Molybdän, Bor, Vanadium, Aluminium und Zirkonium umfassen, in den Oberflächenschichten als härtesteigernde Reaktionsprodukte vorliegen, umfassend Partikel aus wenigstens irgendeinem der Stoffe, die zu der Gruppe von Stoffen gehören, die besteht aus Titannitrid (TiN) und Aluminiumnitrid (AlN), Vanadiumkarbid (VC), Wolframkarbid (WC) und Chromkarbid (Cr₃C₂) und Zirkoniumoxid (ZrO₂), Aluminiumoxid (Al₂O₃) und Chromoxid (Cr₂O₃), **dadurch gekennzeichnet,**
**dass** die harten Oberflächenschichten (6a', 6b') eine zweite, unterliegende Schicht (7a, 7b) bedecken, die Nitride enthält, die in der zweiten Schicht abgeschieden wurden, die Härte in wenigstens einer Zone der zweiten Schicht auf eine Härte erhöhend, die 100 - 1500 HV (Vickershärte) größer ist als die Härte des Stahlmaterials (5) unter der zweiten Schicht, und
**dass** die zweite, unterliegende Schicht (7a, 7b) eine Dicke von wenigstens 0,1 µm (100nm) aber nicht mehr als 10µm (0,010 mm) aufweist,
**dass** die zweiten, unterliegenden Schichten und die harten Oberflächenschichten Mikrostrukturen der Arten aufweisen, die durch Plasmaimmersionsionenimplantation von Stickstoff in das Stahlmaterial und anschließende Plasmaimmersionsionenimplantation von einem oder mehreren der reaktiven Metalle Titan, Wolfram, Chrom, Molybdän, Bor, Vanadium, Aluminium und Zirkonium zusammen mit einem oder mehreren der Elemente Stickstoff, Sauerstoff und Kohlenstoff in den Stahl erhältlich sind,
**dass** der Körper aus einem kaltgewalzten Streifen aus Stahl besteht, der gehärtet und angelassen ist auf eine Härte zwischen 500 und 700 HV (Vickershärte) in seinem gehärteten und angelassenen Zustand, und
**dass** der Körper eine Dicke zwischen 0,07 und 0,6 mm aufweist.

2. Werkzeug gemäß Anspruch 1, **dadurch gekennzeichnet, dass** wenigstens eine Zone der zweiten Schicht eine Härte zwischen 1000 und 3000 HV aufweist.

3. Werkzeug gemäß Anspruch 1, **dadurch gekennzeichnet, dass** die zweite, unterliegende Schicht eine Dicke von wenigstens 0,5 µm (500nm) aber nicht mehr als 10µm (0,010 mm) aufweist.

4. Werkzeug gemäß Anspruch 1, **dadurch gekennzeichnet, dass** die zweite, unterliegende Schicht (7a, 7b) eine Dicke von wenigstens 1 µm (1000nm) aber nicht mehr als 10µm (0,010 mm) aufweist.

5. Werkzeug gemäß Anspruch 1, **dadurch gekennzeichnet, dass** die harten Oberflächenschichten (6a, 6b), die harte Produkte der Reaktion zwischen einerseits einem oder mehreren der Elemente Stickstoff, Sauerstoff und Kohlenstoff und andererseits einem oder mehreren Metallen enthalten, eine Dicke von wenigstens 100 nm (Nanometern), bevorzugt wenigstens 200 nm aufweisen.

6. Werkzeug gemäß Anspruch 5, **dadurch gekennzeichnet, dass** die harten Oberflächenschichten eine Dicke von wenigstens 300 nm aufweisen.

7. Werkzeug gemäß Anspruch 5 und 6, **dadurch gekennzeichnet, dass** wenigstens zwei Metalle in die harten Oberflächenschichten implantiert sind, einschließlich wenigstens eines ersten Metalls, das deutlich leichter ist als ein anderes, zweites Metall, d.h. eine deutlich geringere atomare Zahl als das zweite Metall aufweist, wobei das erste, leichtere Metall in einer äußeren Zone der harten Oberflächenschichten angereichert ist während das zweite, schwerere Metall in einer inneren Zone der harten Oberflächenschichten angereichert ist.

8. Werkzeug gemäß Anspruch 7, **dadurch gekennzeichnet, dass** das erste, leichtere Metall irgendeines der Elemente Bor, Aluminium, Titan, Vanadium und Chrom ist während das das zweite, schwerere Metall irgendeines der Elemente Zirkonium, Niob, Molybdän, Tantal und Wolfram ist.

9. Werkzeug gemäß Anspruch 7 und 8, **dadurch gekennzeichnet, dass** jede Zone eine Dicke von 0,1 - 1 µm aufweist.

10. Werkzeug gemäß irgendeinem der vorstehenden Ansprüche, **dadurch gekennzeichnet, dass** der Stahlkörper des Werkzeugs zwischen den harten Oberflächenschichten eine überwiegend martensitische Struktur aufweist, die größere Aggregationen von abgeschiedenen Zementit und sehr kleine Karbidpartikel enthält.

11. Werkzeug gemäß Anspruch 10, **dadurch gekennzeichnet, dass** das Zementit eine primäre Phase ist, die vor der Plasmaimmersionsionenimplantation abgeschieden wurde während wenigstens ein wesentlicher Abschnitt der sehr kleinen Karbidpartikel durch Transformation von Restaustenit während der Plasmaimmersionsionenimplantation abgeschieden wurden.

12. Verfahren zur Herstellung eines klingenförmigen Werkzeugs (1, 2, 3, 4) gemäß Anspruch 1 durch Bereitstellen eines streifenförmigen Körpers (5) aus einem härtbaren Stahl, der kaltgewalzt, gehärtet und angelassen ist auf eine Härte zwischen 500 und 700 HV (Vickershärte), und weiterhin **dadurch gekennzeichnet,**
**dass** das Werkzeug durch Plasmaimmersionsionenimplantation von Stickstoff ohne irgendein begleitendes Dotierungsmaterial nitriert ist bei einer Temperatur unterhalb der Anlasstemperatur des Stahls, was Abscheidung von Nitriden in einer Schicht (7a, 7b) unterhalb der Oberfläche der flachen Seiten des Werkzeugs verursacht, und
**dass** eine Oberflächenschicht (6a', 6b') anschließend dazu gebracht wird, die nitrierte Schicht (7a, 7b) an den flachen Seiten des Werkzeugs zu bedecken, durch Plasmaimmersionsionenimplantation von einerseits einem oder mehreren der Elemente Stickstoff, Sauerstoff und Kohlenstoff und andererseits einem oder mehreren der Metalle Vanadium, Molybdän, Wolfram, Chrom, Zirkonium, Titan, Bor und Aluminium, wobei die Elemente und Metalle jeweils Reaktionsprodukte in dem Stahl bilden.

13. Verfahren gemäß Anspruch 12, **dadurch gekennzeichnet, dass** die anschließende Plasmaimmersionsionenimplantation bei einer Temperatur von 130°C oder höher durchgeführt wird, jedoch unterhalb der Anlasstemperatur des Stahls, was eine Wärmebehandlung der nitrierten Schicht verursacht für eine solch lange Dauer, dass Stickstoff dazu gebracht wird weiter in den Stahl zu diffundieren und Nitride in einem tieferen Level abgeschieden werden, so dass die unterliegenden Schichten (7a, 7b) daher eine Dicke von wenigstens 0,1 µm (100 nm), bevorzugt wenigstens 0,5 µm (500 nm) und geeigneter Weise wenigstens 1 µm (1000 nm), jedoch nicht mehr als 30% der Dicke des Werkzeugs erhalten.

14. Verfahren gemäß Anspruch 12, **dadurch gekennzeichnet, dass** das Einführen der harten Produkte der Reaktion zwischen einerseits einem oder mehreren der Elemente Stickstoff, Sauerstoff und Kohlenstoff und andererseits einem oder mehreren der Metalle, die die Fähigkeit haben mit einem oder mehreren der Elemente zu reagieren, durch Plasmaimmersionsionenimplantation durchgeführt wird und veranlasst wird für eine so lange Dauer fortzudauern, dass die Reaktionsprodukte, deren Eindringtiefe mit der Dauer zusammenhängt, während der der Plasmaimmersionsionenimplantationsprozess stattfindet, in den Stahl eindringen werden zu einer Tiefe, die der Dicke der Oberflächenschicht von wenigstens 100 nm (Nanometer), bevorzugt wenigstens 200 nm entspricht.

15. Verfahren gemäß Anspruch 12, **dadurch gekennzeichnet, dass**, wenn das Anfangsmaterial für die Plasmaimmersionsionenimplantation ein raffinierter, d.h. niedriglegierter Stahl in Form eines Streifens ist, der kaltgewalzt, gehärtet und angelassen wurde auf eine Härte zwischen 500 und 700 HBV, der Stahlstreifen für wenigstens zehn Stunden in einer Ofenatmosphäre mit einer Temperatur von 130 - 170 °C Plasmaimmersionsionenimplantation ausgesetzt wird.

16. Verfahren gemäß Anspruch 12, **dadurch gekennzeichnet, dass**, wenn das Anfangsmaterial für die Plasmaimmersionsionenimplantation ein legierter, aber kein edler härtbarer Stahl in Form eines Streifens ist, der kaltgewalzt, gehärtet und angelassen wurde auf eine Härte zwischen 500 und 700 HV, der Stahlstreifen für wenigstens zehn Stunden in einer Ofenatmosphäre mit einer Temperatur von 200 - 400 °C Plasmaimmersionsionenimplantation ausgesetzt wird.

17. Verfahren gemäß Anspruch 12, **dadurch gekennzeichnet, dass**, wenn das Anfangsmaterial für die Plasmaimmersionsionenimplantation ein edler härtbarer Chromstahl in Form eines Streifens ist, der kaltgewalzt, gehärtet und angelassen wurde auf eine Härte zwischen 500 und 700 HV, der Stahlstreifen für wenigstens zehn Stunden in einer Ofenatmosphäre mit einer Temperatur von 150 - 300 °C Plasmaimmersionsionenimplantation ausgesetzt wird.

## Revendications

1. Un outil (1, 2, 3, 4) en forme de bande, qui appartient au type d'outils comprenant des lames racleuses et des lames à enduire plates, en forme de bandes, qui présentent un corps (5) en acier d'un type appartenant au groupe des aciers durcis, qui est constitué des aciers au carbone, des aciers faiblement alliés, des aciers alliés mais non inoxydables et des aciers inoxydables martensitiques au chrome, ledit corps ayant
deux côtés plats recouverts d'une couche de surface dure (6a, 6b) qui contient, d'une part, un ou plusieurs des éléments azote, oxygène et carbone, et, d'autre part, un ou plusieurs métaux réactifs avec lesdits éléments, et au moins une partie d'extrémité, qui est la partie de travail de l'outil lors de son utilisation,
lesdits un ou plusieurs des éléments azote, oxygène et carbone, et lesdits un ou plusieurs métaux réactifs, qui comprennent un ou plusieurs métaux parmi le titane, le tungstène, le chrome, le molybdène, le bore, le vanadium, l'aluminium et le zirconium, existent dans lesdites couches de surface en tant que produits de réaction promouvant la dureté comprenant des particules d'au moins l'un quelconque des composés qui appartiennent au groupe de composés consistant en du nitrure de titane (TiN) et du nitrure d'aluminium (AIN), du carbure de vanadium (VC), du carbure de tungstène (WC) et du carbure de chrome (Cr₃C₂), et de l'oxyde de zirconium (ZrO₂), de l'oxyde d'aluminium (Al₂O₃) et de l'oxyde de chrome (Cr₂O₃),
**caractérisé en ce que**
lesdites couches de surface dure (6a', 6b') recouvrent une deuxième couche sous-jacente (7a, 7b) qui contient des nitrures précipités dans ladite deuxième couche, augmentant la dureté dans au moins une zone de ladite deuxième couche à une dureté qui est de 100 à 1500 HV (dureté Vickers) supérieure à la dureté dans le matériau en acier (5) sous ladite deuxième couche, et
ladite deuxième couche sous-jacente (7a, 7b) a une épaisseur d'au moins 0,1 1 µm (100 nm) mais pas supérieure à 10 µm (0,010 mm),
lesdites deuxièmes couches sous-jacentes et lesdites couches de surface dures ont des microstructures des types qui peuvent être obtenus par implantation ionique en immersion plasmatique d'azote dans le matériau d'acier et ensuite implantation ionique en immersion plasmatique d'un ou plusieurs des métaux réactifs dans l'acier tels le titane, le tungstène, le chrome, le molybdène, le bore, le vanadium, l'aluminium et le zirconium avec un ou plusieurs des éléments azote, oxygène et carbone,
le corps se compose d'une bande d'acier laminée à froid qui est durcie et trempée à une dureté comprise entre 500 et 700 HV (dureté Vickers) dans son état trempé et tempéré et
le corps a une épaisseur comprise entre 0,07 et 0,6 mm.

2. Un outil selon la revendication 1, **caractérisé en ce qu'**au moins une zone de ladite deuxième couche a une dureté comprise entre 1000 et 3000 HV.

3. Un outil selon la revendication 1, **caractérisé en ce que** ladite deuxième couche sous-jacente a une épaisseur d'au moins 0,5 µm (500 nm) mais pas supérieure à 10 µm (0,010 mm).

4. Un outil selon la revendication 1, **caractérisé en ce que** ladite deuxième couche sous-jacente (7a, 7b) a une épaisseur d'au moins 1 µm (1000 nm) mais supérieure à 10 µm (0,010 mm).

5. Un outil selon la revendication 1, **caractérisé en ce que** les couches de surface dures (6a, 6b) qui contiennent des produits de réaction durs entre, d'une part, un ou plusieurs des éléments azote, oxygène et carbone, et, d'autre part, un ou plusieurs métaux, présentent une épaisseur d'au moins 100 nm (nanomètres), de préférence d'au moins 200 nm.

6. Un outil selon la revendication 5, **caractérisé en ce que** les couches de surface dures ont une épaisseur d'au moins 300 nm.

7. Un outil selon l'une quelconque des revendications 5 et 6, **caractérisé en ce qu'**au moins deux métaux sont implantés dans les couches de surface dures, comprenant au moins un premier métal sensiblement plus léger qu'un deuxième métal, différent, c'est-à-dire qui a un numéro atomique sensiblement plus faible que le deuxième métal, ledit premier métal plus léger étant enrichi dans une zone extérieure des couches de surface dure, tandis que ledit deuxième métal plus lourd est enrichi dans une zone interne des couches de surface dures.

8. Un outil selon la revendication 7, **caractérisé en ce que** le premier métal plus léger est l'un quelconque des éléments bore, aluminium, titane, vanadium et chrome, tandis que le deuxième métal plus lourd est l'un quelconque des éléments zirconium, niobium, molybdène, tantale et tungstène.

9. Un outil selon l'une quelconque des revendications 7 et 8, **caractérisé en ce que** chaque zone présente une épaisseur allant de 0,1 à 1 µm.

10. Un outil selon l'une quelconque des revendications précédentes, **caractérisé en ce que** le corps en acier de l'outil situé entre les couches de surface dures a une structure majoritairement martensitique, qui contient des agrégats majeurs de cémentite précipitée et de très petites particules de carbure.

11. Un outil selon la revendication 10, **caractérisé en ce que** la cémentite est une phase primaire précipitée préalablement à l'implantation d'ions en immersion plasmatique, tandis qu'au moins une partie substantielle des très petites particules de carbure a été précipitée par transformation d'austénite retenue pendant ladite implantation d'ions en immersion plasmatique.

12. Procédé de fabrication d'un outil (1, 2, 3, 4) en forme de lame selon la revendication 1, **caractérisé par** le fait de prévoir un corps (5) en forme de bande en un acier durcissable qui est laminé à froid, trempé et durci jusqu'à une dureté comprise entre 500 et 700 HV (dureté Vickers), et **caractérisé en outre en ce que**
l'outil est nitruré par implantation d'ions en immersion plasmatique d'azote sans aucun matériau de dopage accompagnant, à une température inférieure à la température de trempe de l'acier, provoquant la précipitation de nitrures dans une couche (7a, 7b) sous les surfaces des faces plates de l'outil, et
une couche de surface (6a', 6b') est ensuite amenée à recouvrir la couche nitrurée (7a, 7b) sur les côtés plats de l'outil par implantation d'ions en immersion plasmatique d'une part d'un ou plusieurs desdits éléments azote, oxygène et carbone, et d'autre part d'un ou plusieurs des métaux vanadium, molybdène, tungstène, chrome, zirconium, titane, bore et aluminium, lesquels éléments et métaux, respectivement, forment des produits de réaction dans l'acier.

13. Procédé selon la revendication 12, **caractérisé en ce que** ladite implantation d'ions en immersion plasmatique subséquente est réalisée à une température de 130 °C ou plus, en étant cependant inférieure à la température de trempe de l'acier, provoquant un traitement thermique de la couche nitrurée pendant une période de temps suffisamment longue pour que l'azote soit amené à se diffuser davantage dans l'acier et que les nitrures soient précipités à un niveau plus profond, de sorte que lesdites couches sous-jacentes (7a, 7b) obtiennent ainsi une épaisseur d'au moins 0,1 µm (100 nm), de préférence au moins 0,5 µm (500 nm) et de manière appropriée au moins 1 µm (1000 nm), mais pas plus de 30% de l'épaisseur de l'outil.

14. Procédé selon la revendication 12, **caractérisé en ce que** l'introduction desdits produits de réaction durs entre, d'une part, un ou plusieurs des éléments azote, oxygène et carbone, et, d'autre part, un ou plusieurs desdits métaux ayant une capacité à réagir avec un ou plusieurs desdits éléments, est réalisée par implantation d'ions en immersion plasmatique et est amenée à se poursuivre pendant une période de temps suffisamment longue pour que les produits de réaction, dont la profondeur de pénétration est liée à la période pendant laquelle l'implantation d'ions en immersion plasmatique se déroule, pénètrent dans l'acier à une profondeur correspondant à l'épaisseur de la couche superficielle d'au moins 100 nm (nanomètre), de préférence d'au moins 200 nm.

15. Procédé selon la revendication 12, **caractérisé en ce que**, lorsque le matériau de départ pour l'implantation d'ions en immersion plasmatique est un acier au carbone raffiné, c'est-à-dire à faible teneur en alliage, sous la forme d'une bande qui a été laminée à froid, trempée et durcie jusqu'à une dureté comprise entre 500 et 700 HBV, la bande d'acier est soumise à une implantation d'ions en immersion plasmatique pendant au moins dix heures dans une atmosphère de four ayant une température de 130 à 170°C.

16. Procédé selon la revendication 12, **caractérisé en ce que**, lorsque le matériau de départ pour l'implantation d'ions en immersion plasmatique est un acier allié durcissable, mais non inoxydable, sous la forme d'une bande laminée à froid, trempée et durcie jusqu'à une dureté comprise entre 500 et 700 HV, la bande d'acier est soumise à une implantation d'ions en immersion plasmatique pendant au moins dix heures dans une atmosphère de four ayant une température de 200 à 400°C.

17. Procédé selon la revendication 12, **caractérisé en ce que** lorsque le matériau de départ pour l'implantation d'ions par immersion plasmatique est un acier au chrome durcissable inoxydable sous la forme d'une bande qui a été laminée à froid, durcie et trempée à une dureté comprise entre 500 et 700 HV, la bande d'acier est soumise à une implantation d'ions en immersion plasmatique pendant au moins dix heures dans une atmosphère de four ayant une température de 150 à 300°C.
